# EUROPEAN PATENT SPECIFICATION

(11) **EP 4 529 835 B1**
(45) Date of publication and mention of the grant of the patent: **11.11.2026**
(21) Application number: 23461655.5
(22) Date of filing: 30.09.2023
(51) Int. Cl.: A61B 5/0507, G01W 1/00, G01K 1/00, G01K 13/20, A61B 5/145, G01K 7/04, G01K 11/00, A61B 5/01

(54) **METHOD OF DETERMINING GLUCOSE CONCENTRATION IN TISSUE, COMPUTER PROGRAM PRODUCT, RADIOMETER AND A SYSTEM THEREFOR**
VERFAHREN ZUR BESTIMMUNG DER GLUKOSEKONZENTRATION IM GEWEBE, COMPUTERPROGRAMMPRODUKT, RADIOMETER UND EIN SYSTEM DAFÜR
MÉTHODE DE DÉTERMINATION DE LA CONCENTRATION DE GLUCOSE DANS UN TISSU, PRODUIT DE PROGRAMME INFORMATIQUE, RADIOMÈTRE ET SYSTÈME CORRESPONDANT

(43) Date of publication of application: 02.04.2025
(73) Proprietor: Medisensonic Spolka Akcyjna, 52-234 Wroclaw (PL)
(72) Inventor: Szczepaniak, Zenon, 26-803 Promna (PL)

(56) References cited:
- WO-A1-2019/147582
- DE-A1- 102006 055 691
- US-A1- 2005 122 254
- US-A1- 2016 051 171

## Description

### Field of the invention

The invention concerns a method for determining the glucose concentration in tissue, a computer program product, a radiometer, and a system for determining the glucose concentration in tissue, especially in the blood.

### State of the art

Determining the blood glucose concentration, i.e., the so-called glycaemia, is important due to the need for monitoring glucose levels related to the treatment and prevention of diabetes.

Diagnosing diabetes based on a blood test gives a precise result. However, for the patient it is associated with a negative stimulus, i.e., pain associated with the invasive examination, so work on alternative solutions is constantly ongoing. This applies especially to mobile devices. Various techniques are used, including iontophoresis -based, ultrasound, detection of respiratory biomarkers, temperature measurement, examination of tissues with electromagnetic waves and light, examination of radiation reflected from tissues, and others.

In the field of measurements with the use of microwave radiometer technology, there are many solutions of radiometer systems for measuring the temperature inside tissues for the diagnosis of cancer, measuring core temperature, diagnosing vesicoureteral reflux, measuring brain temperature, etc.

From the European patent application EP1949084, a device for minimally invasive measurement of the concentration of components contained in the structure of biological tissue is known, the device comprising a microwave energy source generating a range of microwave frequencies, a first antenna connected to the microwave energy source and adapted to transmit at least a portion of the microwave energy to the tissue structure, a second antenna adapted to receive at least a portion of the microwave energy transmitted through the tissue structure, a signal processor adapted to determine a resonant frequency of the received microwave energy, and a data processor adapted to provide an output of the concentration of components in the biological tissue structure on the basis of the determined resonant frequency. US2016051171 discloses a device for interrogating human skin using tight coupling between the transmitter and receiver of the millimeter waves. It uses methods to evaluate changes in the amplitude and/or phase of the transmitted the millimeter waves in order to estimate the blood concentration of glucose. DE102006055691 discloses a device with a diagnostic measuring unit for generating measuring signals, and an evaluating unit for determining a physiological parameter by processing the measuring signals. The measuring unit is integrated in a keyboard of a computer e.g. laptop. The measuring unit is an optical measuring unit for generating oximetric and/or plethysmographic measuring signals. The evaluating unit is provided for determining a local metabolic parameter e.g. local oxygen usage, from the signals of the optical measuring unit. WO2019147582. This document discusses, among other things, systems and methods to compensate for the effects of temperature on sensors, such as analyte sensor. An example method may include determining a temperature-compensated glucose concentration level by receiving a temperature signal indicative of a temperature parameter of an external component, receiving a glucose signal indicative of an *in vivo* glucose concentration level, and determining a compensated glucose concentration level based on the glucose signal, the temperature signal, and a delay parameter.

CN110074790 discloses a method of non-invasive determination of the blood glucose concentration based on a blood layer in an earlobe using a time-domain processed microwave signal. The method includes the following steps: creating an earlobe model; preparing test solutions with different glucose concentrations to simulate blood sugar concentration; placing two antennas on two sides of the earlobe model, where the transmitting antenna emits a high-frequency sinusoidal signal and the receiving antenna receives the sinusoidal signal passing through the simulated earlobe tissues. Using glucose solutions of different concentrations during measurement and signal reception allows one to determine calibration coefficients. The received signals are analyzed in the time domain, wherein the analysis includes filtering the noise from the signals using a wavelet transform algorithm, analyzing the trend of the filtered received signals with the change in glucose concentration, obtaining the rule of the amplitude of the energy density spectrum of the signals after filtering the noise as a function of the glucose concentration value.

Solutions based on exposing the patient to radiation (infrared, light, or radio waves) or ultrasound have the disadvantage that they are not always indifferent to the patient. This especially applies to children under 2 years of age and pregnant women. Occasional exposure does not have a major impact on patients, but constant exposure, such as when the blood glucose concentration is measured using a wearable device, may not be indifferent and verification of the consequences of this exposure requires long-term research.

From the European patent application EP1224905, it is known to use a total power radiometer in temperature measurement, wherein the radiometer operates in the 400 MHz or 100 MHz band at center frequencies of 1.5 GHz or 3.5 GHz and analyses noise radiation generated by tissues to determine temperature.

In the MSc thesis "A Microwave Radiometer System for Use in Biomedical Applications", Laura Ballew, Baylor University, 2006, the use of a microwave radiometer to determine glycemia was proposed. This solution is quite complicated and requires a precise noise source with a stable temperature to obtain a radiometer in the Dicke configuration.

Various radiometer designs are typically used: Total Power Radiometers (TPR), Dicke radiometers, and correlation radiometers.

The disadvantage of TPRs is the sensitivity of the gain to temperature changes. The advantages include simple structure - it is basically a cascade of amplifiers with a detector placed at the end. Its advantage is simplicity, while its disadvantage is susceptibility to changes in the gain and changes in noise characteristics resulting from changes in the temperature of its elements. This is problematic because temperature changes depend not only on the surroundings, but also on the amount of heat released in the elements of the receiving chain due to bias power consumption.

The Dicke radiometer requires the use of additional noise sources with precisely stabilized temperature and microwave components in the form of a circulator and a switch to enable gain control. Such a design ensures insensitivity to changes in the gain and temperature.

The correlation radiometer requires the use of two chains and additional elements in the form of microwave couplers, several detectors (from 4 to 8) and a reference noise source. Such a design ensures insensitivity to changes in the gain. Example of correlation radiometer is disclosed in . Tanner, J. Bosch-Lluis and P. Kangaslahti, "Applications of the Pseudo-Correlation Microwave Radiometer," IGARSS 2022 - 2022 IEEE International Geoscience and Remote Sensing Symposium, Kuala Lumpur, Malaysia, 2022, pp. 7241-7244, doi: 10.1109/IGARSS46834.2022.9883145. Multichannel radiometer with gain control unit for gain control driven by bias current consumption monitoring is disclosed in US2005122254A1.

### Problem to be solved

The problem is to find a passive and non-invasive method for determining the glucose concentration in tissue based on measurement involving a simple apparatus of construction that allows miniaturization - not only portable, but also wearable.

### Summary of the invention

A computer implemented method of non-invasive determination of the blood glucose concentration in the patient's tissue based on a radio noise signal, especially a microwave noise signal, received using an antenna brought close to the patient's skin, according to the invention is distinguished by that the radio noise signal is received using a total power radiometer. Additionally, the method includes the following steps: a step of obtaining transformation coefficients, a step of measuring the temperature of the tissue surface, a step of measuring the temperatures of the active elements of the receiving chain of the radiometer, a step of measuring the currents consumed by the active elements of the receiving chain of the radiometer, a step of measuring the power of the radio noise signal originating from the tissue, and a step of determining the blood glucose concentration in the tissue based on the transformation coefficients and the measured values of the temperature of the tissue surface, the temperatures of active elements of the receiving chain of the radiometer and the currents consumed by the active elements of the receiving chain of the radiometer, and the power of the radio noise signal originating from the tissue. The total power radiometer has an uncomplicated design that is easy to miniaturize. Determining the glucose concentration based on the noise power requires determining transformation coefficients to establish a relationship between the power of the noise generated by the tissue and the effect of the glucose concentration on the antenna mismatch. The coefficients can be determined in reference measurements. In reference measurements, it is also possible to determine the transformation coefficients for the receiving chain if the temperatures of its elements and the currents consumed by them are measured. In this way, it is possible to use a simple, miniaturized design.

Advantageously, the method is implemented in a computer, and in the step of obtaining transformation coefficients, the predefined transformation coefficients are loaded from the computer memory or downloaded from an external server.

Advantageously, the transformation coefficients are determined based on a series of measurements performed on a set of tissues or phantoms with known glucose concentrations, the measurements being performed at different temperatures.

Advantageously, the transformation coefficients are determined in the process of multivariate regression consisting in minimizing the error of matching glucose concentration reference values to the values calculated from the transient characteristics of the radiometer, wherein the variables are: the temperature of the tissue surface, the temperatures of the active elements of the receiving chain of the radiometer, the currents consumed by the active elements of the receiving chain of the radiometer, the power of the radio noise signal. The easiest way is to use the mean squared error criterion.

Advantageously, converting the measured values and the transformation coefficients into the blood glucose concentration includes a step of determining the power of the noise received by the antenna based on the transformation coefficients, the power measured using said radiometer, the temperatures of the active elements of the receiving chain, and the currents consumed by the active elements of the receiving chain of the radiometer. Next, the power of the noise originating from the tissue and reaching the antenna and the reflection coefficient at the interface between the antenna and the tissue is determined using the previously determined power of the noise received by the antenna, the transformation coefficients, and the measured temperature of the tissue surface. Finally, the glucose concentration is determined using the determined reflection coefficient at the interface of the antenna and the tissue and the determined power of the noise originating from the tissue and reaching the antenna.

Advantageously, the transformation coefficients are the weight coefficients of the neural network processing the data set containing the measured temperatures, the currents consumed by the active elements of the receiving chain of the radiometer, and the power of the radio noise signal originating from the tissue.

Advantageously, the measurements of the radio noise signal are carried out in the band ranging from 800 MHz to 24 GHz. Measurements in this frequency range are reliable and have been proven to be repeatable.

Advantageously, the measurement is carried out in a band with a width greater than or equal to 200 MHz, and even more advantageously in a band with a width greater than or equal to 500 MHz. This allows the measurement to be performed faster and the influence of patient movements is eliminated.

A computer program product to be run on a computer adapted to receive measurement data according to the invention comprises instructions that causes the method according to the invention to be performed.

A radiometer according to the invention comprises an antenna and a radio receiving chain with a detector. The antenna is adapted to be applied to the skin of the patient and to receive a radio noise signal from subcutaneous tissue. The radiometer is a total power radiometer equipped with sensors of the temperatures of the active elements of the receiving chain, a sensor of the patient temperature placed on the antenna, and sensors of the currents consumed by the active elements of the receiving chain of the radiometer.

Advantageously, the antenna has a frequency band at least 20% wider than the frequency band of the receiving chain. Owing to this, the device is resistant to the drift of the propagation parameters of the skin and the related shift of the antenna operating band.

Advantageously, the receiving chain of the radiometer contains a cascade of amplifiers separated by isolators.

Advantageously, the receiving chain contains a cascade of amplifiers separated by attenuators with a rated attenuation below 3 dB.

A system for determining glucose concentration based on a radio signal, comprising a processing system and a radiometer with an antenna adapted to be applied to the patient's skin, according to the invention is distinguished by that the radiometer is a radiometer according to the invention, and the processing system connected thereto: sensors of the temperatures of the active elements of the receiving chain of the radiometer, a sensor of the temperature of the tissue surface placed on the antenna, and current measurement modules measuring the currents consumed by the active elements of the receiving chain of the radiometer, and the processing system is adapted to carry out the method according to the invention. Such a system can be miniaturized and can be used as a wearable system while ensuring high accuracy.

### Description of the drawing

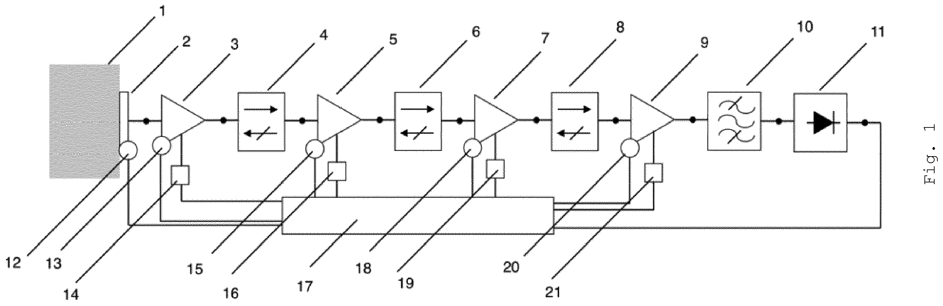

The subject of the invention is described in embodiments in the drawing, in which Fig. 1 shows a block diagram of a system for determining glucose concentration according to the invention.

### Description of embodiments

According to the invention, a passive solution is used. No signal is transmitted to the tissues, but a total power radiometer measures the noise signal originating from the tissues.

The total power radiometer is a radio receiver containing an antenna and a radio receiving chain with a detector shown in the block diagram in Fig. 1. The antenna **2** is adapted to be applied to the skin **1** of the patient and to receive a radio noise signal from subcutaneous tissue.

The antenna is a wideband antenna having the operating band of approximately 1 GHz, which is wider than the operating band of the radiometer chain, which in this example equals 300 MHz. Owing to this, the entire system works even in the presence of a drift of the antenna operating band caused by a change in the electrical parameters of the skin, mainly caused by a change in skin moisture as a result of moisture released due to sweating or TEWL (Transepidermal Water Loss). In this embodiment, antenna **2** is made using not symetric strip - so called microstrip - technology with dimensions and electrical permittivity of the substrate selected to enable the reception of the radiation originating from skin **1.** The microstrip has the advantage that the antenna structure itself shields interfering signals not originating from the patient's skin. A person skilled in the art is also able to use other antenna designs that will ensure the reception of the signal from the tissue.

Antenna **2** is connected directly to the radiometer chain, consisting of a cascade of four amplifiers **3, 5, 7, 9** with a gain of approximately 20 dB, a bandpass filter **10** with a bandwidth of 300 MHz, and a diode detector **11** at the end of the cascade. The output of the detector **11** is the voltage at its terminals representing the measured noise signal power. A person skilled in the art is also able to use other detectors that will ensure determining the signal strength.

Preferably, microwave isolators **4, 6, 8** are placed between the amplifiers. Isolators with an insertion loss of 0.2 dB and an isolation of 20 dB are used. Such a design makes it easier to protect the receiving chain against unwanted oscillations.

The design described above is a typical solution for a total power radiometer and is affected by its typical disadvantages: dependence on the temperature and operating conditions. The influence of these factors is too great to allow measurement of tissue radiation with accuracy sufficient to determine glucose concentration without additional improvements.

The isolators can be replaced with microwave attenuators with a low attenuation, e.g., 2-3 dB. Such a solution is less effective and even increases the influence of chain noise but facilitates miniaturization.

In the present invention, the problem of the influence of the temperature and operating conditions is solved digitally. It was found that the glucose concentration in the tissue affects both the noise generated by the tissue and the electrical parameters of the tissue, which affects the reflectivity at the interface of the tissue and the antenna **2,** as well as the tissue temperature. During the measurement, the antenna reaches a temperature essentially equal to the temperature of the tissue surface. This latter circumstance adds disturbance in the form of the antenna's own noise. Additionally, the measurement performed with the detector **11** is disturbed by the noise of the receiving chain elements, which depends on their temperature and the current they consume. It turned out that the influence of the operating conditions (the temperature and current) of the receiving chain on the received noise signal power can be well modeled - just like the influence of blood glucose on the noise power. It can be done in one process.

To solve the problem of the influence of temperature changes on the gain of the radiometer chain and, further, the influence of these changes on the readings of the power detector **11,** contact temperature sensors (thermocouples) were provided in the following places of the receiving chain:
- antenna - on the ground metalization side, patient temperature sensor **12,**
- amplifiers **3, 5, 7, 9** - on housings, temperature sensors **13, 15, 18, 20,** respectively.

Additionally, sensors **14, 16, 19, 21** for the supply current of individual amplifiers **3, 5, 7, 9,** respectively, were provided.

The signal outputs of the sensors **12, 13, 15, 18, 20 14, 16, 19, 21** and the output of the power detector are wired to the processing system **17,** which contains an analog-to-digital converter and a microprocessor for processing measurement data. The power supply for the radiometer chain elements, the sensors, and the acquisition module is provided by a dedicated power module containing a lithium-ion battery and a set of voltage converters.

A person skill in the art is readily able to propose numerous alternatives to the processing system **17,** including the use of microprocessors, digital signal processors (DSPs), field programmable gate arrays (FPGAs), and application specific integrated circuits (ASICs) for a dedicated signal processing task. Additionally, system-on-chip (SoC) solutions can be adopted, Raspberry Pi or single-board computers, Arduino-based microcontrollers, RISC-V processors, and ARM Cortex-M series microcontrollers have been tested to meet specific computing requirements. In particular, considerations include low-power microcontrollers for battery-powered applications. One can also use distributed solutions and send sensor and detector signals to remote cloud servers for processing, thereby increasing scalability, availability, and data management capabilities.

In this embodiment, the processing system **17** acquires and converts the output signals from the sensors **12, 13, 14, 15, 16, 18, 19, 20, 21** and the detector **11** into the glucose concentration value using the multivariate regression method and predefined transformation coefficients.

The transformation coefficients are determined by performing a series of measurements of phantoms with known glucose concentration at various phantom and ambient temperatures while changing the operating conditions of the receiving chain. These coefficients can also be determined by examining a group of patients in different conditions or even one patient for a long time while changing the operating conditions and regulating the glucose concentration and verifying it in another measurement.

Alternatively, known models can be adopted and their coefficients can be determined. A table approach is also acceptable, in which glycaemia values in tissue 1 are determined during reference measurements based on the adopted model.

Determination of glycemia should be preceded by reference measurements and determination of model coefficients. This can be done in a manner known in the art by measurements performed on simulated phantom tissues with different levels of glucose concentration or patients simultaneously subjected to reference measurement by another method. Measurements performed on many patients or phantoms enable averaging and determining universal transformation coefficients. Measurement performed on a single patient can be used to obtain transformation coefficients dedicated to that specific person and potentially more accurate results.

It is important that the data set obtained from the sensors makes it possible to "extract" the influence of the glucose concentration from other factors affecting the noise power and to determine the transformation coefficients, which enables the determination of glycemia in a measurement that includes not only the noise power, but also the temperature of the tissue surface, the temperatures of the receiving chain elements and the currents consumed by the active elements of the receiving chain. This can be done using multivariate regression or adopting a specific chain model or even using reference data to train a neural network running on the processing system **17,** receiving the data set from the sensors and detector and outputting the glycemia measurement result. In this context, the transformation coefficients are the weight coefficients of the neural network.

The invention can even be implemented by creating a lookup table containing glucose concentration values corresponding to specific sets of measured parameters. The entries of the table then constitute transformation coefficients.

### Industrial applicability and advantages of the invention

As a result of using the solution according to the invention, additional advantages are obtained:
- no need to use additional microwave elements such as input circulator and directional couplers, which facilitates miniaturization,
- single chain instead of double chain,
- no need to use additional reference noise sources, usually with the necessary temperature stabilization,
- no need to use an input switch to switch the input of the amplifying chain successively to the antenna output and noise sources.

The receiver according to the invention can be integrated in clothing and come into contact with the skin of the examined person. It is completely passive and does not emit any measurement signal to the patient's tissues. As a result, it is completely safe and the measurement system and the method according to the invention can be used in the prevention and treatment of diabetes, especially in pregnant women and children under 2 years of age.

## Claims

1. A computer implemented method of non-invasive determination of a blood glucose concentration in a tissue of a patient, the method being based on a radio noise signal received using an antenna kept in a proximity of a skin of the patient, **characterized in that** the radio noise signal is received with a total power radiometer and the method further comprises following steps:
a step of obtaining transformation coefficients,
a step of measuring a surface temperature of the tissue,
a step of measuring temperatures of active elements of a receiving chain of the radiometer,
a step of measuring currents consumed by the active elements of the receiving chain of the radiometer,
a step of measuring a power of the radio noise signal originating from the tissue, a step of determining the blood glucose concentration in the tissue based on
said transformation coefficients and
said measured values of the temperature of the tissue surface and
said temperatures of active elements of the receiving chain of the radiometer and
said currents consumed by the active elements of the receiving chain of the radiometer and
the power of the radio noise signal originating from the tissue.

2. Method according to claim 1, which is implemented in a computer, and in the step of obtaining transformation coefficients, predefined transformation coefficients are loaded from a computer memory or downloaded from an external server.

3. Method according to claim 1, in which the transformation coefficients are determined based on a series of measurements performed on a set of tissues or phantoms of known glucose concentrations, the measurements are being performed at different temperatures.

4. Method according to claim 3, in which the transformation coefficients are determined in the process of multivariate regression consisting in minimizing the error of matching glucose concentration reference values to the values calculated from the transient characteristics of the radiometer, wherein the variables are: the temperature of the tissue surface, the temperatures of the active elements of the receiving chain of the radiometer, the currents consumed by the active elements of the receiving chain of the radiometer, and the power of the radio noise signal.

5. Method according to any of claims from 1 to 4, in which converting the measured values and the transformation coefficients into the blood glucose concentration includes the following steps:
determining the power of the noise received by the antenna based on the transformation coefficients, the power measured using the radiometer, the temperatures of the active elements of the receiving chain of the radiometer, and the currents consumed by the active elements of the receiving chain of the radiometer,
determining the power of the noise originating from the tissue and reaching the antenna and reflection coefficient at the interface between the antenna and the tissue based on the power of the noise received by the antenna, the transformation coefficients, and the temperature of the surface of the tissue,
determining the glucose concentration based on the determined reflection coefficient at the interface of the antenna and the tissue and the determined power of the noise originating from the tissue and reaching the antenna.

6. Method according to claim 1 or 2 or 3, in which the transformation coefficients are weight coefficients of a neural network processing the data set containing
the measured temperature of the tissue surface,
the temperatures of active elements of the receiving chain of the radiometer
the currents consumed by the active elements of the receiving chain of the radiometer, and
the power of the radio noise signal originating from the tissue.

7. Method according to any of claims from 1 to 6, in which the measurements of the radio noise signal are carried out in the band ranging from 800 MHz to 24 GHz.

8. Method according to any of claims from 1 to 7, in which the measurements are carried out in a band with a width greater than or equal to 200 MHz.

9. Method according to claim 8, in which the measurements are carried out in a band with a width greater than or equal to 500 MHz.

10. A radiometer comprising an antenna (**2**) and a radio receiving chain (**3, 4, 5, 6, 7, 8, 9, 10**) with a detector (**11**), the antenna (**2**) being adapted to be applied to a skin (**1**) of a patient and to receive a radio noise signal from a subcutaneous tissue, **characterized in that**
the radiometer is a total power radiometer adapted to measure power of noise signal originating from the tissue equipped with
sensors (**13, 15, 18, 20**) of temperatures of the active elements of the receiving chain of the radiometer,
a sensor (**12**) of a temperature of the tissue surface located on the antenna (**2**),
and sensors (**14, 16, 19, 21**) of the currents consumed by the active elements of the receiving chain of the radiometer.

11. The radiometer according to claim 10, in which the antenna has a frequency band at least 20% wider than the frequency band of the receiving chain.

12. The radiometer according to claim 10, in which the receiving chain contains a cascade of amplifiers (**3, 5, 7, 9**) separated by isolators (**4, 6, 8**).

13. The radiometer according to claim 10, in which the receiving chain contains a cascade of amplifiers (**3, 5, 7, 9**) separated by attenuators with a rated attenuation below 3 dB.

14. A system for determining glucose concentration based on a radio signal, comprising a processing system (**17**) and a radiometer with an antenna (**2**) adapted to be applied to a tissue, **characterized in that** the radiometer is radiometer according to any of claims from 10 to 13, and the processing system (**17**) has connected thereto
sensors (**13, 15, 18, 20**) of the temperatures of the active elements of the receiving chain of the radiometer,
a sensor (**12**) of the temperature of the tissue surface, placed on the antenna (**2**),
current measurement modules (**14, 16, 19, 21**) measuring the currents consumed by the active elements of the receiving chain of the radiometer,
and the processing system (**17**) is adapted to carry out the method as defined in any of claims from 1 to 9.

## Patentansprüche

1. Computerimplementiertes Verfahren zur nicht-invasiven Bestimmung einer Blutzuckerkonzentration in einem Gewebe eines Patienten, wobei das Verfahren auf einem Funkrauschsignal basiert, das unter Verwendung einer in der Nähe einer Haut des Patienten gehaltenen Antenne empfangen wird, **dadurch gekennzeichnet, dass** das Funkrauschsignal mit einem Gesamtleistungsradiometer empfangen wird und das Verfahren ferner folgende Schritte umfasst:
∘ einen Schritt des Erhaltens von Transformationskoeffizienten,
∘ einen Schritt des Messens einer Oberflächentemperatur des Gewebes,
∘ einen Schritt des Messens von Temperaturen von aktiven Elementen einer Empfangskette des Radiometers,
∘ einen Schritt des Messens von Strömen, die von den aktiven Elementen der Empfangskette des Radiometers verbraucht werden,
∘ einen Schritt des Messens einer Leistung des aus dem Gewebe stammenden Funkrauschsignals,
∘ einen Schritt des Bestimmens der Blutzuckerkonzentration im Gewebe basierend auf den Transformationskoeffizienten und den Messwerten der Temperatur der Gewebeoberfläche und den Temperaturen aktiven Elemente der Empfangskette des Radiometers und den von den aktiven Elementen der Empfangskette des Radiometers verbrauchten Strömen und der Leistung des aus dem Gewebe stammenden Funkrauschsignals.

2. Verfahren nach Anspruch 1, welches in einem Computer implementiert ist, und bei dem im Schritt des Erhaltens von Transformationskoeffizienten vordefinierte Transformationskoeffizienten aus einem Computerspeicher geladen oder von einem externen Server heruntergeladen werden.

3. Verfahren nach Anspruch 1, bei dem die Transformationskoeffizienten basierend auf einer Reihe von Messungen bestimmt werden, die an einem Satz von Geweben oder Phantomen mit bekannten Glucosekonzentrationen durchgeführt werden, wobei die Messungen bei unterschiedlichen Temperaturen durchgeführt werden.

4. Verfahren nach Anspruch 3, bei dem die Transformationskoeffizienten im Prozess der multivariaten Regression bestimmt werden, die im Minimieren des Fehlers der Anpassung von Glucosekonzentrations-Referenzwerten an die aus den Übergangscharakteristiken des Radiometers berechneten Werte besteht, wobei die Variablen sind: die Temperatur der Gewebeoberfläche, die Temperaturen der aktiven Elemente der Empfangskette des Radiometers, die von den aktiven Elementen der Empfangskette des Radiometers verbrauchten Ströme und die Leistung des Funkrauschsignals.

5. Verfahren nach einem der Ansprüche 1 bis 4, bei dem das Umwandeln der Messwerte und der Transformationskoeffizienten in die Blutzuckerkonzentration die folgenden Schritte umfasst:
∘ Bestimmen der von der Antenne empfangenen Rauschleistung basierend auf den Transformationskoeffizienten, der unter Verwendung des Radiometers gemessenen Leistung, den Temperaturen der aktiven Elemente der Empfangskette des Radiometers und den von den aktiven Elementen der Empfangskette des Radiometers verbrauchten Strömen,
∘ Bestimmen der Leistung des aus dem Gewebe stammenden und die Antenne erreichenden Rauschens und des Reflexionskoeffizienten an der Grenzfläche zwischen der Antenne und dem Gewebe basierend auf der von der Antenne empfangenen Rauschleistung, den Transformationskoeffizienten und der Temperatur der Oberfläche des Gewebes,
∘ Bestimmen der Glucosekonzentration basierend auf dem bestimmten Reflexionskoeffizienten an der Grenzfläche der Antenne und dem Gewebe und der bestimmten Leistung des aus dem Gewebe stammenden und die Antenne erreichenden Rauschens.

6. Verfahren nach Anspruch 1 oder 2 oder 3, bei dem die Transformationskoeffizienten Gewichtskoeffizienten eines neuronalen Netzwerks sind, das den Datensatz verarbeitet, welcher enthält:
∘ die gemessene Temperatur der Gewebeoberfläche,
∘ die Temperaturen aktiven Elemente der Empfangskette des Radiometers,
∘ die von den aktiven Elementen der Empfangskette des Radiometers verbrauchten Ströme und
∘ die Leistung des aus dem Gewebe stammenden Funkrauschsignals.

7. Verfahren nach einem der Ansprüche 1 bis 6, bei dem die Messungen des Funkrauschsignals in dem Band im Bereich von 800 MHz bis 24 GHz durchgeführt werden.

8. Verfahren nach einem der Ansprüche 1 bis 7, bei dem die Messungen in einem Band mit einer Breite größer oder gleich 200 MHz durchgeführt werden.

9. Verfahren nach Anspruch 8, bei dem die Messungen in einem Band mit einer Breite größer oder gleich 500 MHz durchgeführt werden.

10. Radiometer umfassend eine Antenne (2) und eine Funkempfangskette (3, 4, 5, 6, 7, 8, 9, 10) mit einem Detektor (11), wobei die Antenne (2) angepasst ist, um an eine Haut (1) eines Patienten angelegt zu werden und ein Funkrauschsignal aus einem subkutanen Gewebe zu empfangen, **dadurch gekennzeichnet, dass** das Radiometer ein Gesamtleistungsradiometer ist, das angepasst ist, um die Leistung eines aus dem Gewebe stammenden Rauschsignals zu messen, ausgestattet mit:
∘ Sensoren (13, 15, 18, 20) für Temperaturen der aktiven Elemente der Empfangskette des Radiometers,
∘ einem Sensor (12) für eine Temperatur der Gewebeoberfläche, der sich auf der Antenne (2) befindet,
∘ und Sensoren (14, 16, 19, 21) für die von den aktiven Elementen der Empfangskette des Radiometers verbrauchten Ströme.

11. Radiometer nach Anspruch 10, bei dem die Antenne ein Frequenzband aufweist, das mindestens 20 % breiter ist als das Frequenzband der Empfangskette.

12. Radiometer nach Anspruch 10, bei dem die Empfangskette eine Kaskade von Verstärkern (3, 5, 7, 9) enthält, die durch Isolatoren (4, 6, 8) getrennt sind.

13. Radiometer nach Anspruch 10, bei dem die Empfangskette eine Kaskade von Verstärkern (3, 5, 7, 9) enthält, die durch Dämpfungsglieder mit einer Nenndämpfung unter 3 dB getrennt sind.

14. System zur Bestimmung der Glucosekonzentration basierend auf einem Funksignal, umfassend ein Verarbeitungssystem (17) und ein Radiometer mit einer Antenne (2), die angepasst ist, um an ein Gewebe angelegt zu werden, **dadurch gekennzeichnet, dass** das Radiometer ein Radiometer nach einem der Ansprüche 10 bis 13 ist, und mit dem Verarbeitungssystem (17) verbunden sind:
∘ Sensoren (13, 15, 18, 20) für die Temperaturen der aktiven Elemente der Empfangskette des Radiometers,
∘ ein Sensor (12) für die Temperatur der Gewebeoberfläche, der auf der Antenne (2) platziert ist,
∘ Strommessmodule (14, 16, 19, 21), welche die von den aktiven Elementen der Empfangskette des Radiometers verbrauchten Ströme messen,
∘ und das Verarbeitungssystem (17) angepasst ist, um das Verfahren nach einem der Ansprüche 1 bis 9 auszuführen.

## Revendications

1. Procédé mis en œuvre par ordinateur de détermination non invasive d'une concentration de glucose dans le sang dans un tissu d'un patient, le procédé étant basé sur un signal de bruit radio reçu en utilisant une antenne maintenue à proximité d'une peau du patient , **caractérisé en ce que** le signal de bruit radio est reçu avec un radiomètre à puissance totale et le procédé comprend en outre les étapes suivantes:
∘ une étape d'obtention de coefficients de transformation,
∘ une étape de mesure d'une température de surface du tissu,
∘ une étape de mesure des températures d'éléments actifs d'une chaîne de réception du radiomètre,
∘ une étape de mesure des courants consommés par les éléments actifs de la chaîne de réception du radiomètre,
∘ une étape de mesure d'une puissance du signal de bruit radio provenant du tissu,
∘ une étape de détermination de la concentration de glucose dans le sang dans le tissu sur la base de:
■ lesdits coefficients de transformation et
■ lesdites valeurs mesurées de la température de la surface du tissu et
■ lesdites températures des éléments actifs de la chaîne de réception du radiomètre et
■ lesdits courants consommés par les éléments actifs de la chaîne de réception du radiomètre et
■ la puissance du signal de bruit radio provenant du tissu.

2. Procédé selon la revendication 1, qui est mis en œuvre dans un ordinateur, et dans l'étape d'obtention de coefficients de transformation, des coefficients de transformation prédéfinis sont chargés depuis une mémoire d'ordinateur ou téléchargés depuis un serveur externe.

3. Procédé selon la revendication 1, dans lequel les coefficients de transformation sont déterminés sur la base d'une série de mesures effectuées sur un ensemble de tissus ou de fantômes ayant des concentrations de glucose connues, les mesures étant effectuées à différentes températures.

4. Procédé selon la revendication 3, dans lequel les coefficients de transformation sont déterminés dans le cadre d'un processus de régression multivariée consistant à minimiser l'erreur d'ajustement des valeurs de référence de la concentration de glucose par rapport aux valeurs calculées à partir des caractéristiques transitoires du radiomètre, dans lequel les variables sont : la température de la surface du tissu, les températures des éléments actifs de la chaîne de réception du radiomètre, les courants consommés par les éléments actifs de la chaîne de réception du radiomètre, et la puissance du signal de bruit radio.

5. Procédé selon l'une quelconque des revendications 1 à 4, dans lequel la conversion des valeurs mesurées et des coefficients de transformation en la concentration de glucose dans le sang comprend les étapes suivantes:
∘ la détermination de la puissance du bruit reçu par l'antenne sur la base des coefficients de transformation, de la puissance mesurée en utilisant le radiomètre, des températures des éléments actifs de la chaîne de réception du radiomètre, et des courants consommés par les éléments actifs de la chaîne de réception du radiomètre,
∘ la détermination de la puissance du bruit provenant du tissu et atteignant l'antenne et du coefficient de réflexion à l'interface entre l'antenne et le tissu sur la base de la puissance du bruit reçu par l'antenne, des coefficients de transformation, et de la température de la surface du tissu,
∘ la détermination de la concentration de glucose sur la base du coefficient de réflexion déterminé à l'interface de l'antenne et du tissu et de la puissance déterminée du bruit provenant du tissu et atteignant l'antenne.

6. Procédé selon la revendication 1 ou 2 ou 3, dans lequel les coefficients de transformation sont des coefficients de pondération d'un réseau de neurones traitant l'ensemble de données contenant:
∘ la température mesurée de la surface du tissu,
∘ les températures d'éléments actifs de la chaîne de réception du radiomètre,
∘ les courants consommés par les éléments actifs de la chaîne de réception du radiomètre, et
∘ la puissance du signal de bruit radio provenant du tissu.

7. Procédé selon l'une quelconque des revendications 1 à 6, dans lequel les mesures du signal de bruit radio sont effectuées dans la bande allant de 800 MHz à 24 GHz.

8. Procédé selon l'une quelconque des revendications 1 à 7, dans lequel les mesures sont effectuées dans une bande d'une largeur supérieure ou égale à 200 MHz.

9. Procédé selon la revendication 8, dans lequel les mesures sont effectuées dans une bande d'une largeur supérieure ou égale à 500 MHz.

10. Radiomètre comprenant une antenne (2) et une chaîne de réception radio (3, 4, 5, 6, 7, 8, 9, 10) avec un détecteur (11), l'antenne (2) étant adaptée pour être appliquée sur une peau (1) d'un patient et pour recevoir un signal de bruit radio provenant d'un tissu sous-cutané , **caractérisé en ce que** le radiomètre est un radiomètre à puissance totale adapté pour mesurer la puissance d'un signal de bruit provenant du tissu, équipé de:
∘ capteurs (13, 15, 18, 20) de températures des éléments actifs de la chaîne de réception du radiomètre,
∘ un capteur (12) d'une température de la surface du tissu situé sur l'antenne (2),
∘ et des capteurs (14, 16, 19, 21) des courants consommés par les éléments actifs de la chaîne de réception du radiomètre.

11. Radiomètre selon la revendication 10, dans lequel l'antenne a une bande de fréquences au moins 20 % plus large que la bande de fréquences de la chaîne de réception.

12. Radiomètre selon la revendication 10, dans lequel la chaîne de réception contient une cascade d'amplificateurs (3, 5, 7, 9) séparés par des isolateurs (4, 6, 8).

13. Radiomètre selon la revendication 10, dans lequel la chaîne de réception contient une cascade d'amplificateurs (3, 5, 7, 9) séparés par des atténuateurs ayant une atténuation nominale inférieure à 3 dB.

14. Système de détermination de la concentration de glucose sur la base d'un signal radio, comprenant un système de traitement (17) et un radiomètre avec une antenne (2) adaptée pour être appliquée sur un tissu , **caractérisé en ce que** le radiomètre est un radiomètre selon l'une quelconque des revendications 10 à 13 , et le système de traitement (17) a, connecté à celui-ci:
∘ des capteurs (13, 15, 18, 20) des températures des éléments actifs de la chaîne de réception du radiomètre,
∘ un capteur (12) de la température de la surface du tissu, placé sur l'antenne (2),
∘ des modules de mesure de courant (14, 16, 19, 21) mesurant les courants consommés par les éléments actifs de la chaîne de réception du radiomètre,
∘ et le système de traitement (17) est adapté pour mettre en œuvre le procédé tel que défini dans l'une quelconque des revendications 1 à 9.
